Europäisches Patentamt

European Patent Office

Office européen des brevets

(11) Publication number: **0 405 320 A2**

# EUROPEAN PATENT APPLICATION

(21) Application number: 90111690.5

(22) Date of filing: 20.06.90

(51) Int. Cl.5: **A61M 5/20, A61M 5/28**

(30) Priority: 27.06.89 YU 1306/89
28.12.89 YU 2487/89

(43) Date of publication of application:
**02.01.91 Bulletin 91/01**

(84) Designated Contracting States:
**DE FR GB IT NL SE**

(71) Applicant: ELKOM - TOVARNA STIKALNIH
NAPRAV
Sentiljska c. 49
YU-62000 Maribor(YU)

(72) Inventor: Miroslav, Koren
Belokrajnska 28
Y-62000 Maribor(YU)
Inventor: Bezjak, Marjan, Dipl.-Ing.
Aljazeva 12
Y-62000 Maribor(YU)
Inventor: Vladimir, Vojvodic, Dr.
Bulevar revolucije 237
Y-11000 Beograd(YU)
Inventor: Zlatko, Binenfeld, Dr.
Krasova 14
Y-41000 Zagreb(YU)

(74) Representative: Lewald, Dietrich, Dipl.-Ing.
Patentanwälte Deufel, Schön, Hertel Lewald,
Isartorplatz 6
D-8000 München 2(DE)

(54) **Multipurpose automatic injector.**

(57) The multipurpose automatic injector for automatic injecting one or more different injection substances in liquid or powder aggregation ensures a several years long period of storage of these different and from each other separated substances and, accordingly, it ensures also the required chemical and pharmacological stability of the same. The injector according to this invention is very simple to be handled as there are only two operation steps required for it to be used, namely: to remove the safety cover (51) and place the tapered tip (15) of the barrel (10) to the administering place and press it by force not exceeding 45 N. All the other operations are carried out by the injector fully automatically so that, after the complete removal of the air from the chamber containing the substance in powder (40), it carries out mixing of the separately stored substances (40) and (42) and unlocking of the automatic discharge mechanism. This is followed by the emanation of the injection needle (22) from the barrel (10) and its entering into the body and at the same time injecting the injection solution. The injector is again ready for use after the discharge mechanism is afresh locked and a new barrel (10) is inserted with all its inner members and substances into the cylindrical body (50).

Fig. 1

## MULTIPURPOSE AUTOMATIC INJECTOR

Field of technique to which the present invention belongs

The field of a technique to which the present invention belongs are devices for automatic injecting different liquid substances into a living organism, especially in cases when a human being has to inject a medicament into his own body at a given instant and on conditions not known beforehand. The foreseen classification of the patent subject is according to IPC: A 61M 5/20, A 61M 5/24 and A 61M 5/28 by which devices for injecting medicaments and agents, respectively, into a living organism are characterized, and that in the first place, automatic injecting of different medicaments consisting of two or more liquid or solid components which must be kept separated from each other prior to use of the injector.

State of technique

Devices for manual and automatic injecting two or more component medicament into a living organism are known.
From the Yugoslav Patent Application YU-P-616/88 a multipurpose automatic injecting device is known comprising a housing with an inserted cylindrical body. The housing of the injecting device comprises an inserted self-discharge mechanism containing a safety member, an actuating unit, a piston and a steel compression spring. The cylindrical body comprises a barrel with two or more chambers for storing individual medicament components separated from each other by rubber stoppers. The construction of this known device dictates the procedure of actuating, mixing and injecting in the following consecutive operations: the cylindrical body is turned in the direction from the left to the right (in this instant the liquid component begins to flow from its chamber into another one where the solid component in a powder form is stored and the solid component begins to dissolve) then the device with its tapered part is placed to the administration point, the safety member is removed and the housing of the device is pressed against the body in the place where the injection is to be administered and the discharge button is pressed, (by pressing the button the spring is released and activates the piston which pushes the rubber stoppers and the needle holder which pushes the needle out of the barrel) the injection needle is inserted into the body and the solution is injected into the organism.
The disadvantage and the weak point of this known injector is that prior to its activating five previously necessary consecutive operations must be done what reduces the reliability of its operation and the possibility for rapid use in unexpected and unfavourable conditions.
From EP-A 0 219 899
is known an automatic injector for injecting one or more different injection components. The construction of this known injector has the disadvantage that it does not provide removal of the complete quantity of the air from the chambers in which the substances are stored. As a result of this, the user could inject a substance solution together with the air and the air would enter the blood stream of the user and could cause an air embolism. A further disadvantage of this known injector is that it is intended for a single use only, namely, after the automatic injector has been used the barrel cannot be replaced,what means, that the complete autoinjector shall be thrown away after use. Another further disadvantage of this known device is that the by-pass is represented by a raised peripheral portion of the barrel extending radially outwardly what requires a larger inside diameter of the injector's body (and, consequently, a larger dimension of the automatic injector) and additional cartridge elements enabling a telescoping movement of the barrel in the cylindrical body of the automatic injector. With this known automatic injector a discharge mechanism is not separately shown or described therefore its estimation is impossible.
From EP-A 0144 551
is known a two-component medicament syringe the embodiment of which has the disadvantage that the fabrication of the barrel is very pretentious due to the solution of the by-pass which is projecting outwardly or inwardly in the barrel. Another disadvantage of this known device is that it does not enable removing the air from the storing chambers before injecting the medicament into the body what causes that the user injects the medicament together with the air. If the air enters the blood stream it could cause an air embolism. This known device is intended for a manual operation and has no discharge mechanism.
Regarding the joint disadvantages of the up-to-now known devices for injecting different one-, two- or more component medicaments into the organism which are first of all in the fact that their embodiment conditions the execution of a great number of necessary consecutive operations in the ready-to-use proceeding, in the proceeding of mixing different substances, in the proceeding of discharging a certain mechanism and in the proceeding of injecting the solution into the organism, the further disadvantage is that their construction embodiments do not provide a protection of the barrel tip against the dirtiness, further they do not provide a possibility of repeated applications, they do not enable rapid mixing of a liquid substance with a substance in the powder form. Yet another disadvantage of these known devices is that they do not provide a complete removal of the air from individual chambers in the barrel prior to injecting into the organism what results in injecting a substance solution together with the air from the chambers what could cause an air embolism if the air would enter the blood stream. Yet another disadvantage of these known devices is that their individual safety members which shall be removed prior to use are of very small dimensions what hinders the preparation of the injector for an application in difficult conditions (e.g.: wearing war equipment, gloves..)

Presentation and description of the invention by examples of embodiment and a list of figures

Regarding the preliminary dealing with the state of the technique and the estimation of the state of the technique the disadvantages will be made good by solutions according to our invention which will have the following advantages:

- a simple handling and use of the injector even in most unfavourable conditions,
- small number of necessary operations for the preparation and actuating of the injector,
- the proceeding of mixing a liquid substance and a substance in powder will be carried out within 3-4 seconds,
- quick and complete removal of the air from the chambers prior to injecting the solution into an organism,
- minimal required pushing force for activation of the injector, less than 40 N,
- the barrel tip is protected from dirtiness and eventual contamination by radioactive substances,
- multiple use of the injector body with the mechanism, only the barrel is replaced,
- an automatic activation and operation of the injector,
- a multipurpose use of the injector,
- a simple fabrication and assembly and, consequently, lower production costs,
- a high level of reliability of the injector operation,
- the injector provides a long lasting storing (five years or more) of the present substances in different aggregation, namely, in a condition of their chemical or pharmacological stability.

Previously stated advantages will be reached by a multipurpose automatic injector made according to our invention which comprises a cylindrical body, containing a built-in automatic discharge mechanism and an inserted cylindrical barrel with one, two or more chambers filled with a liquid substance or a substance in powder and in which an injection needle is positioned. Both, the cylindrical body of the injector with the automatic discharge mechanism and the barrel are protected by safety covers.

The invention, as indicated in claims, has the task to provide a completely automatic operation of the discharge mechanism of the automatic injector for injecting one-, two- or more component medicaments which are stored in one, two or more chambers in the barrel by ensuring quick and perfect mixing of the liquid substance and a substance in powder providing a necessary concentration of the dissolved medicament and the solution, respectively, to ensure furthermore the entire removal of the air from the chambers in the barrel and to ensure, as well, injecting of the whole quantity of the medicament solution into the organism at any time and in all, even most difficult conditions.

In continuation, the invention will be presented more in detail on base of the embodiment example and the accompanying drawings from which the figures illustrate the following:

Fig. 1 longitudinal sectional view of the autoinjector according to the present invention in a condition in which it can be transported and in the position before use;

Fig. 2 longitudinal sectional view of the automatic injector according to the present invention, shown in Fig. 1, with a removed safety cover and in the initial operation of use, in the initial proceeding of mixing the liquid substance with the substance in powder;

Fig. 3 longitudinal sectional view of the automatic injector as shown in Fig. 2, in the position after the completed proceeding of mixing the liquid substance with the substance in powder;

Fig. 4 longitudinal sectional view of the automatic injector as shown in Fig. 3, in the position after the operation of the automatic discharging of the mechanism and after the completed proceeding of injecting the solution;

Fig. 5 longitudinal sectional view of the automatic injector as shown in Fig. 4, in position after use, with the safety cover put on again and in the ready-to-be-transported position;

Fig. 6 cross-sectional view I - I of the automatic injector without inside members, in the sphere of the by-pass channels, shown in Fig. 1;

Fig. 7 longitudinal sectional view II - II of the barrel of the automatic injector, drawn without inside members, shown in Fig. 1.

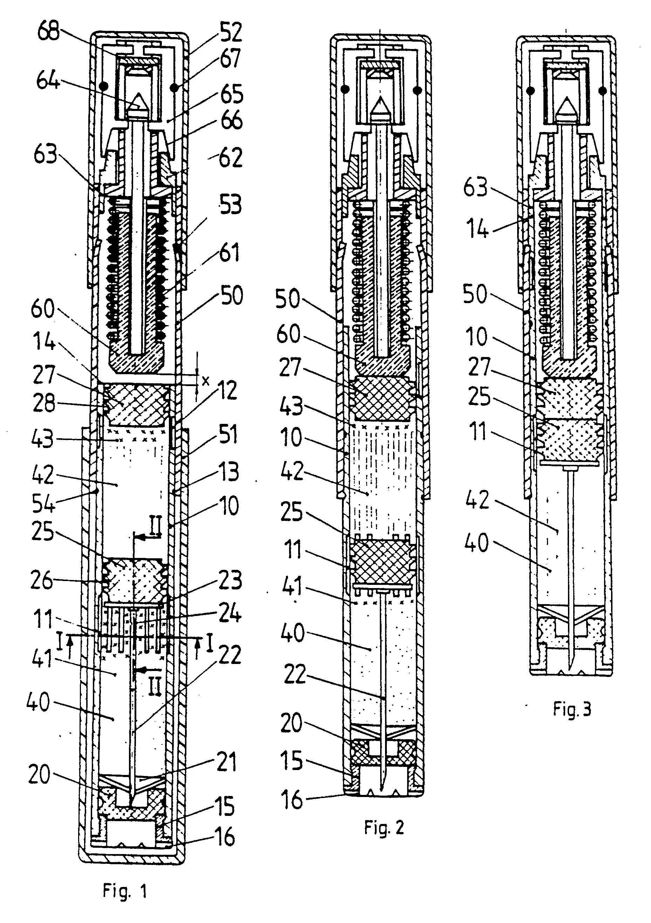

Fig. 1 illustrates a multipurpose automatic injector according to the present invention containing a cylindrical body 50 with two catches 53 in its wall lying in the face to face position under the angle of 180° and with two semicircular ridges 54 at the front end of the cylindrical body 50 projecting inwardly in the face to face position under the angle of 180°.

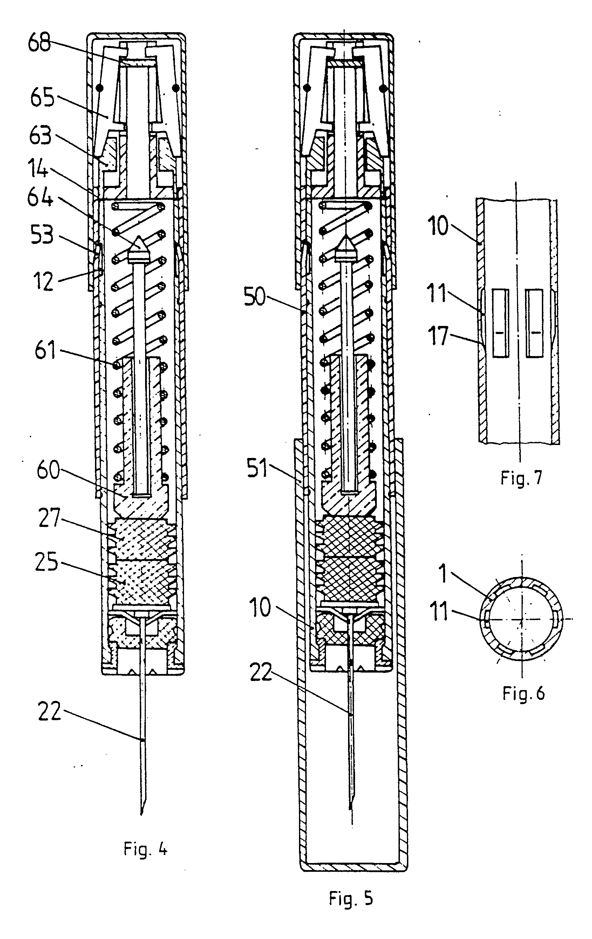

Further the automatic injector according to the present invention comprises a barrel 10 which is telescopically inserted in the cylindrical body 50, its position being determined by two semicircular ridges 54 when they jump into the groove 13. The barrel 10 has under the edge 14 of its outer wall two locking grooves 12 which are in mutual position under the angle of 180°. The middle part of the barrel 10 has on its inside wall longitudinally positioned by-pass channels 11 which are in detail shown by sections I-I and II-II in Figs. 6 and 7. The distal end of the barrel 10 is closed by the barrel tip 15 with the aeration channels 16 being pushed into the inside of the barrel 10. On the barrel tip 15 is adjoined the sealing stopper 20 with a dead aperture into which extends the sharp distal end of the injection needle 22 which is centrically guided by a needle guide 21. The injection needle 22 with a duct 24 is pressed in the cetring boring of the needle holder 23 which is accommodated above the by-pass channels 11. The needle holder 23 with its rear face adjoins the chamber separating stopper 25 which has trapezium-shaped ribs 28. The sealing piston 27 adjoins with its rear face the edge 14 of the barrel 10. Between the sealing piston 27 and the chamber separating stopper 25 the barrel 10 accommodates a stored liquid substance 42 while in the chamber between the separating stopper 25 and the sealing stopper 20 the second substance 40 in powder form is stored. The barrel 10 which is inserted in the cylindrical body 50 is covered with a safety cover 51 whose inner wall adjoins the outer wall of the cylindrical body 50.

Furthermore, the present automatic injector according to the invention comprises an automatic discharge mechanism which is accommodated inside the cylindrical body 50 and comprises a spring giude 60 with a coiled compression spring 61 which by its rear end engages the bottom of the cylindrical body 50. On the tapering part of the cylindrical body 50 from the outside an unlocking ring 62 with two feelers 63 is positioned. In the longitudinal slot in the outer wall of the tapered portion of the cylindrical body 50 two unlocking lamellas 65 are inserted whose inner slope 66 adjoins the outer slope of the unlocking ring 62 while the top legs adjoin the plate 68. The bottom legs on the unlocking lamellas 65 extend into the inside of the tapered part of the cylindrical body 50 and on these two legs presses the bottom side of the anchor head 64. The anchor 64 is firmly inserted inside the spring guide 60. The unlocking lamellas 65 are bounded together by an elastic ring 67. The automatic discharge mechanism is covered by a cover 52 which by its inner wall firmly adjoins the outer wall of the cylindrical body 50. Figs. 6 and 7 show the arrangement and the form of the by-pass channels 11 which are positioned longitudinally on the inner wall of the barrel 10 and are evenly disposed on the inner circumference. The by-pass channels 11 have at the side of the chamber with a substance in powder 40, shown in Fig. 1, an outletting slope 17. The by-pass channels are provided in such a manner that their length is substantially longer than the length of the separating stopper 25, shown in Fig. 1.

Figs. 2, 3 and 4 illustrate individual consecutive operations of work of the automatic injector according to the present invention which will be presented in detail also by Fig. 1 and Fig. 5.

Fig. 1 shows the automatic injector before use where the position of the barrel 10 is determined by a snap connection of the groove 13 in the outer sleeve of the barrel 10 with the semicircular ridge 54 on the outer wall of the cylindrical body 50. In this position of the barrel 10, between the edge 14 on the barrel rear end and the bottom side of the spring guide head 60 an air gap X is formed, the compression spring 61 on the spring guide being in a compressed position. The automatic discharge mechanism is now in its ready-to-use form.

After the safety cover 51 is removed the tapered tip 15 of the barrel is placed to the administering point. By pressing the cover 52 the pressure force is transferred to the cylindrical body 50, the barrel 10 moves telescopically into the cylindrical body 50, on its way it moves along the length of the air gap X, releases the snap connection of the groove 13 and the semicircular ridge 54, the head of the spring guide 60 being moved up to the sealing piston 27.

Fig. 2 illustrates the automatic injector in the initial proceeding of mixing of the liquid substance 42 with the substance in powder 40. By the further un interrupted action of the force of pressure, described in Fig. 1, the force is being transferred still further to the cylindrical body 50 and causes a further telescopical movement of the barrel 10 into the cylindrical body 50, the head of the spring guide 60 adjoining the sealing piston 27 and preventing the movement of the sealing piston 27 in the direction of the automatic discharge mechanism. In the phase of the telescopical movement of the barrel 10 into the cylindrical body 50, the volume of the chamber,retaining the liquid substance 42 and the air cushion 43, gets reduced at a part of the volume of the air cushion 43 due to the compressibility of the air. The overpressure occurred in the volume reduced chamber, retaining the liquid substance 42, acts upon the separating stopper 25 and moves it in the direction to the sealing stopper 20 what is enabled by the air cushion 41 in the chamber retaining the substance in powder 40. The injection needle 22 which is centrically guided by the needle guide 21, pierces the wall in the dead aperture in the sealing stopper 20 and, consequently, the air of the air cushion 41 can escape through the injection needle 22 into the inner space of the barrel tip 15 and through the aeration channels 16 outwards. Due to the escape of the air of the air cushion 41 and acting of the pressure via the liquid in the chamber containing the liquid substance 42, the separating stopper 25 moves forward to the sphere of the by-pass channels 11, the by-pass channels open and the liquid substance begins to flow intensively through the by-pass channels into the chamber with the substance in powder 40, what can be seen in Fig. 2. Due to the evenly disposed by-pass channels 11 in a circumferentially manner in the inner wall of the barrel 10 and due to the outlet slope 17 of the outlet side of the by-pass channels 11, what is shown in Figs. 6 and 7, the proceeding of intensive mixing of the liquid substance 42 with the powder substance 40 begins. Within all the time of the mixing action the aeration runs in the compartment in which the injecting solution is being formed. According to the said above an entire removal of the air is reached and, as shown in Fig. 3, the air is prevented to be injected into the human organism.

Fig. 3 shows that the edge 14 of the barrel 10, after the proceeding of mixing the liquid 42 and the powder 40 substances is finished, pushes the feelers 63 on the unlocking ring 62 upwards. The slope on the outer circumference of the unlocking ring 62 acts upon the slope 66 on the leg of the unlocking lamellas 65 which are bounded together by the elastic ring 67. The anchor head 64 gets released and under the influence of the precharged compression spring 61 on the spring guide 60 it acts upon the sealing piston 27 and via the sealing piston upon the separating stopper 25 and the needle holder 23 with the needle 22. The injection needle 22 begins to move forward out of the barrel 10 and during the whole movement of the injection needle 22 the injecting of the injection solution is being accomplished into the tissue of the organism as shown in Fig. 3.

Fig. 4 shows that the catches 53 get locked in the locking grooves 12 at the instant when the barrel 10 moves in its final position. Thus, the return hit due to the action of the compression spring 61 in the hand of the user of the automatic injector is prevented. In the described final position of the barrel 10 the latter can be removed from the automatic injector in such a manner that it is turned by approximately 90° and then drawn out of the cylindrical body 50. In the reverse turn of operations a new filled barrel 10 can be inserted into the automatic injector.

Fig. 5 illustrates the automatic injector after the completed phase of injecting the injection solution into the organism, the barrel 10 being covered with a safety cover 51 which is pulled on the cylindrical body 50. The automatic injector is now ready for transportation.

Through all the operation stages of the automatic injector the pressure force must not exceed 45 N what can be performed by the automatic injector according to the invention whose construction embodiment ensures movement of the previously described parts in the barrel 10 without forces for overcoming the friction being mathematically summed up. This is enabled by the solutions of the air gap X, the air cushion 43 and the air cushion 41 and performable through the trapezium-shaped ribs 28 and 26 on the sealing piston 27 and on the separating stopper 25 which are both inserted in the barrel 10 in such a manner that the slope of the ribs 28 and 26 is always turned in the direction of the by-pass channels 11.

## Claims

1. The multipurpose automatic injector for injecting one substance or the solution of more substances in a liquid or a powder aggregation comprises a combination of a substantially cylindrical body containing an discharge mechanism composed of more members and containing a single-chamber or multi-chamber barrel in wich a movable sealing piston is provided which closes said barrel; accommodated in the barrel is a movable separating stopper circumferentially adjoining the inner wall of the barrel in a sealing manner and, consequently, separating the substances from each other so that they can be kept stored for a longer period of time not to lose their chemical and pharmacological stability; furthermore, accommodated in the barrel is an injection needle with a needle holder and a needle guide, as well as a barrel tip which closes the barrel; and that said injector comprises a safety cover pulled on the cylindrical body and a cover of the automatic discharge mechanism firmly attached to the cylindrical body; characterized in that approximately one quarter of the length of the barrel (10) is telescopically inserted in the inside of the cylindrical body (50) the position of the barrel (10) being precisely determined by a semicircular ridge (54) on the inner wall of the cylindrical body (50) when it jumps into the groove (13) in the outer wall of the barrel (10), a part of the barrel (10) outside the cylindrical body (50) being protected by a safety cover. (51).

2. The multipurpose automatic injector according to claim 1, characterized in that the barrel (10) is constructed in a form of a proper hollow cylinder without bulges on outer or inner walls, with a reduced inner diameter at the front face of the barrel and made from polypropilene resistant to injection means.

3. The multipurpose automatic injector according to claims 1 and 2, characterized in that in the inner wall of the barrel (10) three or more longitudinal mutually parallel, circumferentially disposed by-pass channels (11) are provided so that they are recessed inside the inner wall of the barrel (10) and have their outlet slope (17) carried out in the direction of the reduced inner diameter of the wall of the barrel (10), the length of the by-pass channels being longer than the length of the separating stopper (25).

4. The multipurpose automatic injector according to claims 1-3, characterized in that the barrel (10) has in its outer wall, between the edge (14) and the groove (13) two locking grooves (12) with an arc length 140° and mutually shifted axes of symmetry by 180°.

5. The multipurpose automatic injector according to claims 1-4, characterized in that inserted in the barrel (10) is a hollow tip (15) of the barrel accommodated in the sphere of its reduced inner diameter, the barrel tip (15) being carried out so that it has on its front plane four or more recessed aeration channels (16).

6. The multipurpose automatic injector according to claims 1-5, characterized in that the barrel (10) comprises above the by-pass channels a movable separating stopper (25) whose circumference adjoins the inner wall of the barrel (10) in a sealing manner, and that, prior to use of the injector closes the chamber containing the substance in powder (40), thereby keeping it separated from the chamber containing the liquid substance (42); adjoining with its front face the needle holder (23) with the injection needle (22) and that the separating stopper (25) has ribs (26) on the outer circumference whose cross-section has a form of an irregular trapezium and which are with their slopes turned in the direction of operation.

7. The multipurpose automatic injector according to claims 1,2 and 6, being characterized in that the injection needle (22) is sealingly gripped by the needle holder (23) and they both are accommodated in the middle of the chamber containing the substance in powder (40), in the barrel (10), between the separating stopper (25) and the sealing stopper (20) in such a manner that the injection needle (22) is guided by the needle guide (21) and that the tip of the injection needle (22) is positioned in the dead aperture of the sealing stopper (20) and the entering boring into the injection needle (22) is carried out through the needle holder (23).

8. The multipurpose automatic injector according to claim 1, being characterized in that in the wall before the tapering of the cylindrical body (50) two partially movable catches (53) are provided whose free ends extend into the inside of the cylindrical body (50) and that have their arc length 40° and mutually shifted axes of symmetry by 180°.

9. The multipurpose automatic injector according to claims 1 and 8, being characterized in that in the middle of the spring guide (60) the anchor (64) is firmly inserted which with its cone-shaped head extends into the tapered portion of the cylindrical body (50).

10. The multipurpose automatic injector according to claims 1, 8 and 9, being characterized in that on the outer wall of the tapered portion of the cylindrical body (50) an unlocking ring (62) is positioned with its outer slope and the feelers (63) extending into the inside of the cylindrical body (50).

11. The multipurpose automatic injector according to claims 1 and 8-10, being characterized in that in the longitudinal slot in the outer wall of the tapered portion of the cylindrical body (50) two unlocking lamellas (65) are inserted with the top legs extending into the tapered portion of the cylindrical body (50) and adjoining the plate (68) and with the middle leg exteding under the head of the anchor (64) while the lower legs with their slope (66) adjoin the slope of the unlocking ring (62).

12. The multipurpose automatic injector according to claims 1 and 8-11, being characterized in that the unlocking lamellas (65) are encircled and bounded together by the elastic ring (67).

13. The multipurpose automatic injector according to claims 1-12, being characterized in that on the outer wall of the cylindrical body (50) over the barrel (10) the safety cover (51) is placed.

14. The multipurpose automatic injector according to claims 1-13, being characterized in that prior to use the user shall remove the safety cover (51), place the tapered tip (15) of the barrel (10) with its aeration channels (16) to the administering place and press continuously the injector by hand, the injection needle (22) piercing the wall of the dead aperture on the sealing stopper (20) resulting in the beginning of the aeration of the chamber, containing the substance in powder (40), through the injection needle (22) and the aeration channels (16) and in the beginning of mixing the liquid substance (42) with the substance in powder (40) through the by-pass channels (11).

15. The multipurpose automatic injector according to claim 14, being characterized in that after the process of mixing being completed, the discharge mechanism gets automatically unlocked and acts via the sealing piston (27) and the separating stopper (25) upon the injection needle (22) and pushes it forward out of the barrel (10) into the organism simultaneously injecting the injection solution and, that after the injecting action carried out, the safety cover (51) shall be put on again.

16. The multipurpose automatic injector according to claims 1-15, being characterized in that in the injector the barrel (10) with all its inner members can be changed in such a manner that the barrel (10) is turned by 90° and drawn out.

Fig. 1

Fig. 2

Fig. 3

Fig. 4

Fig. 5

Fig. 7

Fig. 6